(19) Europäisches Patentamt
European Patent Office
Office européen des brevets

(11) Publication number: **0 007 128**
**B1**

(12) # EUROPEAN PATENT SPECIFICATION

(45) Date of publication of patent specification: **16.02.83**

(51) Int. Cl.³: **C 07 D 209/52**

(21) Application number: **79200347.7**

(22) Date of filing: **27.06.79**

(54) **Derivatives of 3-azabicyclo(3.1.0)hexane and processes for their preparation.**

(30) Priority: **06.07.78 US 922407**

(43) Date of publication of application:
**23.01.80 Bulletin 80/2**

(45) Publication of the grant of the patent:
**16.02.83 Bulletin 83/7**

(84) Designated Contracting States:
**BE CH DE FR GB IT NL SE**

(56) References cited:
**DD - A - 133 943**
**DE - A - 2 646 188**
**US - A - 4 088 652**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Kollmeyer, Willy Dietrich**
**1008 Stratford Lane**
**Modesto, California 95350 (US)**

(74) Representative: **Rogers, Roy Charles et al,**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 007 128 B1

Derivatives of 3-azabicyclo[3.1.0]hexane and processes for their preparation

This invention relates to derivatives of 3-azabicyclo[3.1.0]hexane and processes for their preparation.

2-Carboxy-3-azabicyclo[3.1.0]hexane and certain of its salts and esters have very valuable biological properties being capable of sterilizing the male anthers of plants. In the past, the 3-azabicyclohexane ring structure has been, however, extremely difficult to synthesize.

According to the disclosure of our copending European Patent Application No. 79200348.5, 2-carboxy-3-azabicyclo[3.1.0]hexane can be readily prepared from 3-azabicyclo[3.1.0]hexane in a stepwise synthesis proceeding through 3-azabicyclo[3.1.0]hex-2-ene formed by sequential halogenation and dehydrohalogenation of the saturated starting material, said 3-azabicyclo[3.1.0]hex-2-ene being subsequently converted to 2-cyano-3-azabicyclo[3.1.0]hexane via a bisulphite adduct and the 2-cyano-3-azabicyclo[3.1.0]hexane being hydrolyzed or alcholized to the desired 2-carboxy-3-azabicyclo[3.1.0]hexane product. In the present invention an attractive route has been found to a novel compound, 3-benzyl-3-azabicyclo[3.1.0]hexane which, in turn, can be directly converted to 3-azabicyclo[3.1.0]hexane, the starting material for the above described synthesis of 2-carboxy-3-azabicyclo-[3.1.0]hexane by catalytic hydrogenolysis.

U.S. Patent 4,088,652 discloses a synthetic technique for converting certain 1-aryl-1,2-cyclopropanedicarboximides into 1-aryl-3-benzyl-3-azabicyclo[3.1.0]hexane. However, the process disclosed in this reference uses a substituted cyclopropanecarboximide starting material which is first reduced and then reacted with a benzoyl chloride or acid anhydride to form a N-acyl compound which is subjected to a second reduction to afford the desired 1-aryl-3-benzyl-3-azabicyclo[3.1.0]hexane. With the present invention, the novel 3-benzyl-3-azabicyclo[3.1.0]hexane can be prepared from readily available starting materials using a simpler two step synthetic technique.

The invention therefore provides the novel compound 3-benzyl-3-azabicyclo[3.1.0]hexane which has the formula

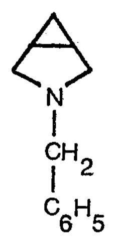

(I)

The invention also provides a process for the preparation of this compound, which comprises reacting the compound 3-benzyl-3-azabicyclo[3.1.0]hexan-2,4-dione, which has the formula

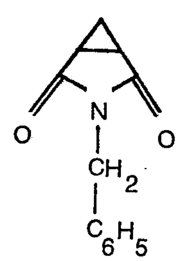

(II)

with a complex aluminium hydride reducing agent, thereby selectively reducing the carbonyl groups. The compound II is also a novel compound.

Preferred reducing agents are lithium aluminium anhydride and, especially, sodium bis-(2-methoxyethoxy)-aluminium dihydride or sodium aluminium diethyl dihydride.

Suitably an excess of the aluminium hydride reducing agent is used. Depending on the specific reducing agent, an excess of up to six-fold, preferably up to two-fold, may be used. Preferably from 10 to 40%, for example 20 to 40% excess is used. Any suitable solvent, for example an aromatic hydrocarbon, such as toluene, or an ether, may be used. The hydride reducing agents are often marketed as solutions or dispersions in liquid hydrocarbons. Such products can be used directly, the hydrocarbon not interfering with the desired reaction, or the hydrocarbon may be removed. The reduction is preferably effected by mixing the reactants and heating the stirred mixture to a moderately elevated temperature, for example 50°C to 120°C. If preferred, the reactants can be mixed initially at a low temperature, for example 0 to 15°C, and then heated together. The resulting mixture is then suitably treated with water or an aqueous solution of an alkali metal base to destroy the excess reducing agent, and is filtered to remove inorganic salts and stripped of solvent to yield the crude desired product, from which the pure product can be isolated by conventional techniques. Alternatively, a phase separation step may be useful.

The compound of the general formula II is prepared by reacting cis 1,2-cyclopropane dicarboxylic acid or the anhydride thereof, with benzylamine, and the invention therefore also provides this novel process. Preferably the acid or anydride is treated with a substantially equimolar amount of benzylamine, at a moderately elevated temperature, for example at a temperature of from 150°C to 200°C. If desired, the reaction may be carried out in the presence of a suitable solvent, for example water or in the absence of a solvent, water being distilled off from the reaction mixture during the course of the reaction. Preferably the reaction is carried out in the presence of a solvent which is an azeotroping agent, so that water may be removed during the reaction. Xylene is especially suitable. The product can, if desired, be recovered from the final reaction mixture by any suitable method, for example by removing any water which has not been distilled off during the course of the treatment, and pouring the crude product (ordinarily an oil) into a suitable liquid medium, for example isopropyl alcohol, in which the desired product is at most only sparingly soluble, but which is a solvent for any unreacted material and any by-products. Alternatively, the product can be further reacted *in situ*.

If free cis 1,2-cyclopropane dicarboxylic acid, rather than its anhydride, is used as starting material, it may be admixture with the trans acid without any adverse affect on the course of the reaction, since the trans acid forms a polymer which can be removed at any convenient stage in the reaction. A preferred procedure involves dehydrating the mixed cis and trans acids to form the cyclic cis anhydride and the polymeric trans anhydride, and then distilling off the cis anhydride to use in the subsequent reaction.

1,2-Cyclopropane dicarboxylic acid and its anhydride can be prepared by treatment of a diester of the acid, as described in Journal of the American Chemical Society, Vol. 80, pages 6568—6572 (1958).

The compound of the general formula I is converted into 3-azabicyclo[3.1.0]hexane, which has the formula

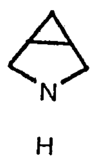

(III)

by hydrogenolysis of the N-benzyl bond. This hydrogenolysis is suitably effected by catalytic hydrogenation using a noble metal preferably a palladium catalyst. The benzyl compound is hydrogenolyzed under mild conditions, preferably using a palladium-on-carbon catalyst. Room temperature, for example a temperature up to 50°C, or somewhat above, is generally suitable. Suitable hydrogen pressures are in the range of from 245 to 3038 kPa (1.5 to 30), for example 343 to 784 kPa (2.5 to 7 atmospheres gauge). Low pressures, for example 1.5 to 2 atmospheres gauges, may be very suitable. Suitable solvents include the lower alkanols, for example ethanol or, especially, methanol.

Isolation of the 3-azabicyclo[3.1.0]hexane may be facilitated by converting it to its hydrochloride salt, which is nonvolatile.

As mentioned previously, 3-azabicyclo[3.1.0]hexane may be converted into 2-carboxy-3-azabicyclo[3.1.0]hexane using the process described in our co-pending application No. 79200348.5.

The following Examples illustrate the invention. In these Examples, the identities of intermediate and final products were confirmed by appropriate chemical and spectral analysis.

## Example 1

*(A) Preparation of (cis,trans)-1,2-cyclopropanecarboxylic acid* (1)

With exclusion of moisture, a stirred mixture of 43.3 g (1.01 mol.) of 56% sodium hydride-mineral oil dispersion in 200 ml of toluene was treated with 10—20 ml of a blend of 100.1 g (1 mol.) ethyl acrylate and 122.6 g (1 mol.) ethyl chloroacetate followed by several drops of ethanol. After an induction period of about 1 hour, steady gas and heat evolution began. Then the remaining mixed ester reagent was carefully added dropwise with ice-bath cooling so as to maintain a reaction temperature of 30—38°C. After addition was completed (4 hours), the mixture was cooled, washed with water, and dried ($MgSO_4$). Distillation gave diethyl (cis,trans)-1,2-cyclopropanedicarboxylate, (1A), as a colourless liquid, b.p.: 78—89°C (93.1 Pa) (Literature value: 50—90°C (133 Pa)).

Specification of 190.1 g (1.02 mol.) of *1A* was achieved with 116.0 g (2.90 mol.) of sodium hydroxide in 780 ml water at reflux for 5 hours. After removal of ethanol with a rotary evaporator, the

remaining solution was acidified with a slight excess of 12N hydrochloric acid (268 ml, 3.2 mol.). The resulting mixture was stripped to dryness. The solid residue was extracted with hot ethyl acetate (3×500 ml). Evaporation of solvent from a dried ($MgSO_4$) extracts gave *1*, m.p.: 108—128°C (with gas evolution).

*(B) 3-Benzyl-3-azabicyclo[3.1.0]hexane-2,4-dione* (2)

A mixture of 128.1 g (0.984 mol.) of *1*, 100 ml water, and 105.5 g (0.984 mol.) of benzylamine was heated at 180°C, for 2 hours, while water was allowed to distil out. The mixture then was cooled somewhat and the warm mixture was slowly poured into 1000 ml of isopropyl alcohol. The mixture was thoroughly chilled and filtered to give impure *2*, m.p.: 90—150°C. The major by-product appears to be a non-cyclic material formed from the trans-isomer of the acid. It is not very readily separated from *2*. It does not, however, interfere in the subsequent reduction of *2*, but forms a by-product from which *3* is readily separated (step C(i)).

*(C) 3-Benzyl-3-azabicyclo[3.1.0]hexane* (3)

(i) 142.3 g (0.707 mol.) of the product of step B were added, in portions, to an ice-cooled and stirred suspension of 105.6 g (1.59 mol.) of 57.2% aluminium hydride/mineral oil disperion in 2000 ml of tetrahydrofuran, the temperature of the mixture being maintained at or below 15°C. The mixture was cautiously brought to reflux temperature and refluxed for 4 hours, and was then allowed to stand overnight at room temperature. Then, 200 ml of 50% sodium hydroxide solution, followed by 150 ml of water, were added over a 3 hour period, Celite was added and the mixture was filtered to remove inorganic salts. The filtrate was dried ($MgSO_4$), the solvent was evaporated, and the residue was distilled in the presence of several drops of Dow Corning Antifoam A (registered Trade Mark), to give *3*, b.p: 73—74 (1.33 Pa).

(ii) 63 g (0.486 mol.) of *1* were added in portions of 150 ml of thionyl chloride. The mixture was refluxed for 1 hour, then was stripped. The residue was distilled to give cis-1,2-cyclopropanedicarboxylic acid anhydride (*3*(i)), b.p.: 134°C (1333 Pa). 2.14 g (0.02 mol) of benzylamine were carefully added to 2.24 g (0.02 mol.) of *3*(i); the reaction was very exothermic. The mixture was heated at 180°C for 2 hours. After cooling, the residue was recrystallised from isopropyl alcohol to give 3-benzyl-3-azabicyclo[3.1.0]hexane-2,4-dione, as white needles, m.p.: 90—91°C (*3*(ii)). A cooled (0°C) solution of 305 ml (1.09 mol.) of 70% sodium bis(2-methoxyethoxy)aluminium hydride in benzene diluted with 600 ml of ether was treated with 48.7 g (0.25 mol.) of *3*(ii). The mixture was stirred at 0° for $\frac{1}{2}$ hour and refluxed for 3 hours. After standing at room temperature overnight, excess metal hydride was destroyed by cautiously adding cold water. The mixture was filtered after addition of diatomaceous silica (Celite). The ether layer was removed and the aqueous layer was extracted with ether. The combined organic layers were dried ($MgSO_4$). Ether and 2-methoxy-ethanol were evaporated under reduced pressure to give *3*, as an oil. Distillation gave *3*, b.p.: 78—80°C (1.33 Pa).

*(D) 3-Azabicyclo[3.1.0]hexane* (4)

Catalytic hydrogenation (2.05 g, 10% palladium on charcoal) of 40.5 g (0.234 mol.) of undistilled *3*, procedure (ii), in 150 ml ethanol was carried out with a Parr apparatus (53 gk Pa) (4$\frac{1}{2}$ atmospheres *3*, procedure (ii), in 150 ml ethanol was carried out with a Parr apparatus (53 kg Pa) (4$\frac{1}{2}$ atmospheres gauge initially, room temperature) overnight. After filtration of catalyst, ethanol was fractionally distilled fractions were collected: b.p.: 104—110°C, 7.78 g, purity 84% and b.p.: 110—114°C, 9.00 g, purity 94%. The relative amounts of *4* and impurity ethanol in these fractions were determined by NMR analysis.

*(E) 3-Azabicyclo[3.1.0]hexane hydrochloride* (5)

82.6 g (0.476 mol.) of *3*, procedure (i), in 100 ml of absolute alcohol, were catalytically hydrogenated (4 g, 10% palladium in charcoal) in a Parr apparatus for 5 days at room temperature, the pressure of the hydrogen being maintained at 4$\frac{1}{2}$ atomospheres gauge. The catalyst was removed, 39.7 ml (0.476 mol.) of concentrated hydrochloric acid were added and the solution was concentrated under reduced pressure. The last traces of water and ethanol were removed by azeotropic distillation with benzene to give *5*, m.p.: 158—161°C. An analytical sample, m.p.: 161—163°C (with gas evolution), was obtained by trituration of the above product with cold isopropyl alcohol.

Example 2

*(A) Cis- and trans-diethylcyclopropane 1,2-dicarboxylates*

A 50 litre glass reactor equipped with a stirrer, addition funnel, thermowell and condenser was blanketed with nitrogen. Sodium hydride (6360 g, of 50% in oil, 132.5 M) was charged and washed three times with dry toluene (5 litres, 2$\frac{1}{2}$ litres, 3 litres), the solvent being successively removed with a filter stick. Addition of dry toluene (6 litres) was followed by a portion (1 litre) of a mixture of ethyl chloroacetate (16.2 kg, 132.5 M) ethyl acrylate (13.25 kg, 132.5 M, stabilized with 0.02% hydroquinone) and dry toluene (12 litres). The slurry was stirred and absolute ethanol (3 ml) added as initiator to minimize the induction period. Reaction commenced after about an hour. Cooling with

isopropanol/solid carbon dioxide was applied lowering the internal temperature to 10°C. The addition of mixed esters-toluene feedstock was then continued at such a rate as to maintain a pot temperature in the range 10—20°C, with the cooling bath at −20 to 40°C. The overall addition time was 11½ hours and reaction was essentially complete after a further 2 hours.

After stirring overnight the contents were cooled to 5°C and a solution of glacial acetic acid (1.5 litre, 26 M) in toluene (3 litres) slowly added, bringing the pH to approximately 6. This mixture was then slowly added with vigorous agitation to a 100 litre vessel containing water (12.5 litres) cracked ice (10 kg) and concentrated hydrochloric acid (4.5 litres).

High acidity was maintained throughout this operation. A further portion of water 25 l was used for transfer purposes. Phases were separated and the organic layer washed with demineralized water (10 litres). The combined aqueous solution was re-extracted with toluene (5 litres). Toluene was removed on rotary evaporator at 60°/3325 Pa (25mm Hg) to give crude product (27.9 kg, *cis/trans* ratio 75/25).

Claisen distillation afforded mixed *cis-* and *trans*-diethyl cyclopropane 1,2-dicarboxylates, b.p.: 75—90° at 66.5—133 Pa (0.5—1 mm Hg) (18.08 kg, *cis/trans* = 72/28); yield = 73% based on starting esters.

*(B) Cyclopropane 1,2-dicarboxylic anhydride* (6)

Mixed diethylcyclopropane 1,2-dicarboxylate (28.3 kg, *cis/trans* 72/28, 152.2 M), formic acid (70 litres of 98%, 1818 M) and concentrated sulphuric acid (610 ml of 98%, 11.2 M) were brought to reflux with stirring in a 100 litre glass reactor. Ethyl formate was distilled off. The reaction was shut down and allowed to cool overnight. Sodium formate (1.7 kg, 25 M) was added to neutralize sulphuric acid catalyst and formic acid removed *in vacuo* by rotary evaporation; the crude mixed acids containing sodium sulphate weighed 22.7 kg at this stage. Crude acids were dissolved in acetic anhydride (40 litres) at 40—50° and dehydrative cyclization effected by addition of this solution to stirred acetic anhydride (10 litres) maintained at 135°. This required 1½ hours and reaction was 'finished' for a further 1 hour at 125—140°. After cooling to 20°, insoluble material (sodium sulphate) was removed by filtration through a Celite pad. Acetic acid and excess acetic anhydride were removed by rotary evaporation *in vacuo.*

Claisen distillation, after removal of light and afforded *6*, b.p. 105—120° 26.6 to 133 Pa at {0.2 to 1.0 mm Hg} (10.4 kg, 92,9 M) which readily crystallized on cooling, m.p.: 55—60°. The yield over the acidolysis and dehydration steps was 85% based on *cis*-content of the cyclopropane di-ester. Still bottoms weighed 5.19 kg and consist of polymer *trans*-anhydride.

*(C) 3-Benzyl-3-azabicyclo[3.1.0]hexane-2,4-dione* (7)

A 10 litre stirred reaction flask was charged with *6* (3.37 kg, 30.1 M) and the temperature raised to 75°. Heating was discontinued and benzylamine (3.22 kg, 30.1 M) was added cautiously. The temperature rose over one hour to 160° at which point 2 kg has been fed. Heating was resumed to maintain 155—165° whilst removing water in a gentle current of nitrogen via a Dean-Stark trap.

Benzylamine addition was complete after 2 1/4 hours, and the reaction was 'finished' at 175—180° for a further 1½ hours. The reactor contents were allowed to cool to 110° and added with stirring to isopropanol (12.5 litres). After cooling to 10°, product was recovered by filtration. Air drying gave 7, (5.44 kg, 27.1 M), m.p. 93—95°, which represents a 90% yield on *6*.

*(D) 3-Benzyl-3-azabicyclo[3.1.0]hexane* (8)

A 250 litre anchor stirred glass-lined reactor was purged and blanketed with nitrogen. Sodium bis-(2-methoxyethoxy)aluminium dihydride (63 kg, 61.5 litres of 70% w/v in toluene, 212.5 M) and dry (azeotropically) toluene (25 litres) were then charged and brought to 60°. 7 (17.1 kg, 85 M) was dissolved in dry toluene at 60° (85 litres) and added over a period of 3½ hours to the reducing agent with no external heating. The temperature rose from 60° to 110° during this time. Reaction was 'finished' for ½ hour at 110°. After cooling to 10°, a solution of sodium hydroxide (8.5 kg, 212.5 M) in water (80 litres) was run in slowly. After the first 1.5 litre had been added, hydrogen evolution ceased and the temperature was easily controlled at 15—25° by water cooling; total caustic addition time was 1½ hours.

The organic layer was washed with demineralized water (30 litres) and the combined aqueous layers re-extracted with toluene (10 litres).

Removal of solvent in a rotary evaporator gave crude produce (15.48 kg).

Claisen distillation afforded *8*, b.p. 95—100°C at 26.6—66.5 Pa {0.2—0.5 mm Hg} (13.57 kg, 78.4 M) in 92% yield.

*(E) 3-Azabicyclo[3.1.0]hexane* (9)

5% Palladium on carbon (250 g) was slurried with methanol (1.2 litres) which has been cooled to −70°. *8* (2.6 kg, 15.0 M) the catalyst slurry and an additional portion of methanol (4 litres) were loaded into a 10 litre anchor stirred stainless steel autoclave. The vessel was closed, purged three times with nitrogen, twice with hydrogen and finally to 1862 kPa (18 atomospheres gauge) with hydrogen. After 4

hours the pressure was boosted to 2842 kPa (28 atmospheres gauge) and maintained at this value overnight. After 21 hours the conversion of starting material was 95%. A further 4 hours reaction (25 hours total) resulted in complete disappearance of *8*.

Catalyst was removed by filtration and washed with methanol. Toluene, methanol azeotrope was distilled off. *9* was obtained in 95% yield. A nitrogen blanket was maintained throughout.

**Claims**

1. 3-benzyl-3-azabicyclo[3.1.0]hexane.

2. A process for the preparation of the compound claimed in claim 1, characterized in that it comprises a) reacting cis 1,2-cyclopropane dicarboxylic acid or the anhydride thereof with benzylamine to afford 3-benzyl-3-azabicyclo[3.1.0]hexan-2,4-dione and b) reacting the 3-benzyl-3-azabicyclo[3.1.0]hexane-2,4-dione produced in step a) with a complex aluminium hydride reducing agent, thereby selectively reducing the carbonyl groups to yield the desired 3-benzyl-3-azabicyclo[3.1.0]hexane.

3. A process as claimed in claim 2, characterized in that the reducing agent is lithium aluminium hydride, sodium bis-(2-methoxyethoxy)-aluminium dihydride or sodium aluminium diethyl dihydride.

4. A process as claimed in either claim 2 or claim 3, characterized in that step b) is performed at a temperature in the range of from 50 to 120°C.

5. A process as claimed in any one of claims 2 to 4 characterized in that in step a) the benzylamine and the cyclopropane derivative are heated together at a temperature in the range of from 150 to 200°C, and water is distilled off during the reaction.

6. A process as claimed in any one of claims 2 to 5, characterized in that it comprises the further step of converting the 3-benzyl-3-azabicyclo[3.1.0]hexane into 3-azabicyclo[3.1.0]hexane by hydrogenolysis of the N-benzyl bond.

7. A process as claimed in claim 6, characterized in that the hydrogenolysis is carried out using gaseous hydrogen and a palladium catalyst.

**Revendications**

1. 3-benzyl-3-azabicyclo[3.1.0]hexane.

2. Procédé de préparation du composé revendiqué dans la revendication 1, caractérisé en ce qu'il implique a) de faire réagir l'acide cis-1,2-cyclopropane-dicarboxylique ou son anhydride avec de la benzylamine pour donner la 3-benzyl-3-azabicyclo[3.1.0]hexane-2,4-dione et b) de faire réagir la 3-benzyl-3-azabicyclo[3.1.0]hexane-2,4-dione produite dans l'étape a) avec un agent réducteur hydrure d'aluminium complexe, réduisant ainsi de façon sélective les groupes carbonyle pour donner le 3-benzyl-3-azabicyclo[3.1.0]hexane désiré.

3. Procédé selon la revendication 2, caractérisé en ce que l'agent réducteur est l'hydrure de lithiumaluminium, le dihydrure de sodium bis-(2-méthoxyéthoxy)-aluminium ou le diéthyl-dihydrure de sodium-aluminium.

4. Procédé selon la revendication 2 ou la revendication 3, caractérisé en ce qu'on conduit l'étape b) à une température située dans un intervalle de 50 à 120°C.

5. Procédé selon l'une quelconque des revendications 2 à 4 caractérisé en ce que dans l'étape a) on chauffe ensemble la benzylamine et le dérivé de cyclopropane à une température située dans un intervalle de 150 à 200°C, et en ce qu'on enlève l'eau par distillation pendant la réaction.

6. Procédé selon l'une quelconque des revendications 2 à 5, caractérisé en ce qu'il comprend l'étape supplémentaire consistant à transformer le 3-benzyl-3-azabicyclo[3.1.0]hexane en 3-azabicyclo[3.1.0]hexane par hydrogénolyse de la liaison N-benzyle.

7. Procédé selon la revendication 6, caractérisé en ce qu'on conduit l'hydrogénolyse en utilisant de l'hydrogène gazeux et un catalyseur au palladium.

**Patentansprüche**

1. 3-Benzyl-3-azabicyclo[3.1.0]hexan.

2. Verfahren zur Herstellung der Verbindung nach Anspruch 1, dadurch gekennzeichnet, daß es die folgenden Stufen umfaßt:
a) Umsetzung von cis-1,2-Cyclopropandicarbonsäure oder dem Anhydrid davon mit Benzylamin unter Bildung von 3-Benzyl-3-azabicyclo[3.1.0]hexan-2,4-dion und
b) Umsetzung des in Stufe 1 gebildeten 3-Benzyl-3-azabicyclo[3.1.0]hexan-2,4-dions mit einem komplexen Aluminiumhydrid-Reduktionsmittel, wodurch die Carbonylgruppen selektiv reduziert werden unter Bildung des gewünschten 3-Benzyl-3-azabicyclo[3.1.0]hexans.

3. Verfahren nach Anspruch 2, dadurch gekennzeichnet, daß das Reduktionsmittel Lithiumaluminiumhydrid, Natrium-bis-(2-methoxyethoxy)aluminiumhydrid oder Natriumaluminiumdiethyldihydrid ist.

4. Verfahren nach einem der Ansprüche 2 oder 3, dadurch gekennzeichnet, daß die Stufe b) bei

einer Temperatur im Bereich von 50 bis 120°C durchgeführt wird.

5. Verfahren nach einem der Ansprüche 2 bis 4, dadurch gekennzeichnet, daß in Stufe a) das Benzylamin und das Cyclopropanderivat zusammen auf eine Temperatur im Bereich von 150 bis 200°C erhitzt werden und, das Wasser während der Reaktion abdestilliert wird.

6. Verfahren nach einem der Ansprüche 2 bis 5, dadurch gekennzeichnet, daß es die weitere Stufe der Umwandlung des 3-Benzyl-3-azabicyclo[3.1.0]hexans in 3-Azabicyclo[3.1.0]hexan durch Hydrogenolyse der N-Benzylbindung umfaßt.

7. Verfahren nach Anspruch 6, dadurch gekennzeichnet, daß die Hydrogenolyse unter Verwendung von gasförmigem Wasserstoff und einem Palladiumkatalysator durchgeführt wird.